# EUROPEAN PATENT APPLICATION

(11) **EP 4 231 004 A1**
(43) Date of publication of application: **23.08.2023**
(21) Application number: 22157823.0
(22) Date of filing: 21.02.2022
(51) Int. Cl.: G01N 27/04, G01N 33/46

(54) **ELECTRODE DEVICE FOR A RESISTIVE MEASUREMENT OF A MOISTURE CONTENT OF A CONSTRUCTION MATERIAL**

(71) Applicant: Technische Universität München, 80333 München (DE)
(72) Inventor: Aondio, Patrik, 86899 Landsberg am Lech (DE); Ott, Stephan, 90607 Rückersdorf (DE); Kamml, Michael, 81371 München (DE)
(74) Representative: Lucke, Andreas

(57) **Abstract**

An electrode device for measuring a resistivity of a surrounding medium comprises a rod, a first terminal element and a second terminal element, and a plurality of electrode elements. The rod is elongated along a longitudinal direction. The first terminal element and the second terminal element are arranged on the rod at different positions along the longitudinal direction. The electrode elements are electrically insulated from each other, and the plurality of electrode elements comprises a deformable electrode element. The deformable electrode element is arranged on the rod at a position along the longitudinal direction between the first terminal element and the second terminal element. The deformable electrode element comprises an electrically conductive surface facing away from the rod with a transverse distance from the rod. The first terminal element and the second terminal element are adapted to apply a compressive force along the longitudinal direction to the deformable electrode element to increase the transverse distance of the electrically conductive surface of the deformable electrode element from the rod.

## Description

### TECHNICAL FIELD

The disclosure relates to a device for a resistive measurement of a moisture content of a construction material, in particular to an electrode device, in particular to an electrode device for determining a depth profile or depth gradient of the moisture content.

### BACKGROUND

The moisture content of a construction material such as concrete or wood has a critical impact on the mechanical properties and the lifetime of the construction material. For example, a change in the moisture content may give rise to expansion or shrinkage of the construction material. An excessive moisture content may result in corrosion or rotting. Therefore, monitoring the moisture content of the construction material of a building gives valuable information about its lifetime or the risk of a failure, respectively. Moreover, monitoring the moisture content prior to or during the construction of the building, for example to reach an optimum moisture content in a drying process of the construction material, may improve the quality and durability of the building.

The moisture content of the construction material may be determined using a resistive measurement. For this purpose, screw, bolt or nail electrodes can be inserted into the construction material through a surface thereof. Subsequently, an electrical resistance between the screw, bolt or nail electrodes is measured. When the arrangement of the screw, bolt or nail electrodes is known, the moisture content may be determined based on the measured resistance and using a reference model or table.

Therefore, the arrangement of the screw, bolt or nail electrodes needs to be controlled precisely when inserting them into the construction material or measured precisely thereafter.

The reference model or table to determine the moisture content based on the measured resistance may not only depend on the arrangement of the screw, bolt or nail electrodes, but also on the temperature of the construction material. Therefore, a thermometer may be placed at a position at the surface of the construction material to measure a temperature at this position. The reference model or table may be adjusted using the measured temperature. CN 202442985 U describes a conventional electrode for a resistive moisture measurement.

### OVERVIEW

In view of the technical problems laid out above, there is a need for an improved device for determining a moisture content of a construction material based on a measurement of its resistivity. This objective is achieved with an electrode device according to claim 1. Claim 11 provides a method for mounting an electrode device in a surrounding medium. The dependent claims relate to preferred embodiments.

In a first aspect, an electrode device for measuring a resistivity of a surrounding medium comprises a rod, a first terminal element and a second terminal element, and a plurality of electrode elements. The rod is elongated along a longitudinal direction. The first terminal element and the second terminal element are arranged on the rod at different positions along the longitudinal direction. The electrode elements are electrically insulated from each other. The plurality of electrode elements comprises a deformable electrode element. The deformable electrode element is arranged on the rod at a position along the longitudinal direction between the first terminal element and the second terminal element. The deformable electrode element comprises an electrically conductive surface facing away from the rod with a transverse distance from the rod. The first terminal element and the second terminal element are adapted to apply a compressive force along the longitudinal direction to the deformable electrode element to increase the transverse distance of the electrically conductive surface of the deformable electrode element from the rod.

In a conventional resistive measurement of the moisture content, the arrangement of the screw, bolt or nail electrodes may change over time, for example when the construction material shrinks or expands. This may happen as a consequence of a change in the moisture content. In particular, a contact resistance between a screw, bolt or nail electrode and the construction material may change over time. This may significantly affect the measured resistance and cause a significant error of the determined moisture content. The problem is particularly pronounced if the construction material is wood.

In the electrode device according to the present invention, the transverse distance of the electrically conductive surface of the deformable electrode element from the rod may be increased (or decreased) to improve an electrical contact between the electrically conductive surface and the surrounding medium. The improved electrical contact improves the reliability and accuracy of the resistivity measurement and consequently of a measurement of the moisture content of the surrounding medium. In particular, increasing or decreasing the transverse distance may be applied to compensate a shrinkage or an expansion of the surrounding medium, for example a shrinkage or an expansion related to a change of the moisture content of the surrounding medium. Therefore, the compressive force along the longitudinal direction may be adjusted when a change in a contact resistance between the electrically conductive surface and the surrounding medium is detected or when a shrinkage or an expansion of the surrounding medium is detected. Alternatively, or in addition, the first terminal element and the second terminal element may be used to apply a bias compressive force along the longitudinal direction to account for a shrinkage or an expansion of the surrounding medium that may happen in the future.

The resistive measurement of the moisture content may use the deformable electrode element in combination with a second electrically conductive portion of the electrode device, such as one of the terminal elements, the rod, or a further electrode element which is electrically insulated from the deformable electrode element by an insulation element.

Preferably, the electrode device comprises a plurality of deformable electrode elements and the resistive measurement of the moisture content of the surrounding medium may be performed using an electrode device combining a plurality of deformable electrode elements and at least one insulation element arranged between a pair of deformable electrode elements, which will be described in the following.

In particular, the plurality of electrode elements may comprise a plurality of deformable electrode elements arranged on the rod at different positions along the longitudinal direction between the first terminal element and the second terminal element, wherein each deformable electrode element of the plurality of deformable electrode elements comprises an electrically conductive surface facing away from the rod, with a transverse distance from the rod.

The electrode device may further comprise an insulation element arranged between a pair of deformable electrode elements of the plurality of deformable electrode elements. The insulation element may be adapted to electrically insulate the deformable electrode elements of the pair from each other.

A compressive force applied by the first and second terminal elements along the longitudinal direction to the deformable electrode elements may then increase the transverse distances of the respective electrically conductive surfaces of the deformable electrode elements from the rod.

The resistivity measurement according to this embodiment, using the electrode device with a plurality of deformable electrode elements, may therefore benefit from an improved electrical contact between the electrically conductive surfaces and the surrounding medium for each electrode, further improving the reliability and accuracy of the resistivity measurement.

In a conventional resistive measurement of the moisture content, the information depth of the resistance measurement and of the determined moisture content depends on the arrangement of the screw, bolt or nail electrodes, and in particular on the depth at which they are inserted into the construction material. Typically, the lateral spacing of the screw, bolt or nail electrodes along the surface exceeds this depth, resulting in a measurement of the moisture content at the surface of the construction material. A measurement of the moisture content deeper inside the construction material or even of a depth profile or of a depth gradient of the moisture content might give additional or more relevant information about the construction material, for example in terms of lifetime, risk of failure, quality, and/or durability.

The electrode device according to the description may provide a well-defined and/or predefined arrangement of the deformable electrode elements, in particular with respect to their spacing. This may avoid errors in the measurement of the moisture content of the surrounding medium related to uncertainties in the arrangement of the electrodes.

The arrangement of the deformable electrode elements along the longitudinal direction may result in a measurement of the resistivity along said longitudinal direction. In a typical measurement, the longitudinal direction may correspond to a direction perpendicular to a surface of the surrounding medium, or to a depth direction of the surrounding medium, respectively. By providing the electrode device with a suitable length and/or suitable positions of the deformable electrode elements along the longitudinal direction between the first terminal element and the second terminal element, the electrode device may be adapted for measuring the moisture content at a well-defined depth of the surrounding medium, e. g. from a surface. In particular, the positions of the deformable electrode elements along the longitudinal direction may be adapted to provide a sufficient depth (exceeding, for example, 1 cm, 2 cm, or 3 cm, depending on the material) to measure a bulk moisture content of the surrounding medium rather than a surface or near-surface moisture content.

Each deformable electrode element of the plurality of deformable electrode elements may comprise a substantially identical transverse distance between its respective electrically conductive surface and the rod, for example prior to, after, and/or during applying the compressive force along the longitudinal direction.

The transverse distance may be a distance along a transverse direction, which may be perpendicular to the longitudinal direction.

The first terminal element and the second terminal element may be adapted to apply the compressive force along the longitudinal direction to the deformable electrode element to compress the deformable electrode element along the longitudinal direction and to expand the deformable electrode element along the transverse direction to increase the transverse distance.

The first terminal element and the second terminal element may be adapted to apply the compressive force along the longitudinal direction to each deformable electrode element of the plurality of deformable electrode elements to compress the deformable electrode elements along the longitudinal direction and to expand the deformable electrode elements along the transverse direction to increase the transverse distance.

Materials commonly applied in the deformable electrode element may essentially maintain their volume upon applying the compressive force along the longitudinal direction. Therefore, compressing the deformable electrode element along the longitudinal direction may result in an expansion along the transverse direction, which may be used to increase the transverse distance. In other words, the electrode device with the rod, the first terminal element, the second terminal element, and the deformable electrode element(s) may deflect the compressive force along the longitudinal direction to increase the transverse distance.

The deformable electrode element(s) may have a circular or annular cross section. In particular, the deformable electrode element(s) may be arranged such that the rod is in a center of the circular/annular cross section and/or the transverse distance may refer to a difference of radii of the rod and the circular/annular cross section.

Alternatively, or in addition, the transverse distance may correspond to a maximum transverse distance of the electrically conductive surface from the rod.

A corresponding embodiment may be well-adapted to a geometry of a round (in cross-sectional view), for example drilled, hole or opening in the surrounding medium for inserting the electrode device.

The deformable electrode element(s) may encircle the rod in a plane perpendicular to the longitudinal direction fully or along at least three directions, wherein each of the at least three directions is perpendicular to at least another one of the at least three directions.

A corresponding shape of the deformable electrode element(s) may facilitate arranging the deformable electrode element(s) on the rod and fixing its/their transverse position(s) relative to the rod, for example when inserting the electrode device into the surrounding medium.

The rod may comprise a thread, and the first terminal element and/or the second terminal element may comprise a counter-thread adapted to mount the first terminal element and/or the second terminal element to the rod moveably along the longitudinal direction.

The thread and the counter-thread may be adapted to adjust the position of the first terminal element and/or of the second terminal element on the rod along the longitudinal direction.

In particular, the compressive force along the longitudinal direction may be applied using the thread and the counter-thread.

The thread and the counter-thread may provide reliable and economical means to provide or adjust the compressive force along the longitudinal direction.

The electrode device may comprise a plurality of insulation elements comprising the insulation element, wherein each insulation element of the plurality of insulation elements is arranged between a pair of deformable electrode elements, in particular a pair of neighboring deformable electrode elements. Each insulation element may be adapted to electrically insulate the deformable electrode elements of the respective pair from each other.

Preferably, each insulation element of the plurality of insulation elements is arranged between a different pair of deformable electrode elements.

The pair of deformable electrode elements may be a pair of neighboring deformable electrode elements of the plurality of deformable electrode elements, although the resistivity may in principle be measured between each pair of deformable electrode elements.

In embodiments with a plurality of insulation elements, resistivities and hence moisture contents of the surrounding medium may be measured for a plurality of positions along the longitudinal direction, and consequently for a plurality of depths in the surrounding medium. For example, the plurality of depths may be related to positions of the insulation elements along the longitudinal direction or to positions of pairs of deformable electrode elements along the longitudinal direction.

In such embodiments, the electrode device may be applied to generate a depth profile or a depth gradient of the moisture content of the surrounding medium, for example instead of providing a measure of the moisture content at a single depth.

The deformable electrode element(s) may be adapted to provide a larger deformation than the first terminal element and/or the second terminal element and/or the insulation element(s) when the compressive force is applied along the longitudinal direction.

The larger deformation may refer to a larger compression along the longitudinal direction and/or to a larger expansion along the transverse direction. In other words, the deformable electrode element(s) may have a lower Young's modulus or Poisson's ratio than the first terminal element and/or the second terminal element and/or the insulation element(s) with respect to a compressive force along the longitudinal direction.

The deformable electrode element(s) may comprise or be an elastic element. The deformable electrode elements may each comprise or be an elastic element.

The elastic element(s) may be adapted to, when the compressive force along the longitudinal direction is applied, compress along the longitudinal direction and to expand along the transverse direction to increase the transverse distance.

The elastic element(s) may be adapted to provide an initial shape prior to applying the compressive force along the longitudinal direction, and may be adapted to essentially recover the initial shape when the compressive force along the longitudinal direction is released.

According to embodiments, the elastic element(s) may be arranged between the rod and the electrically conductive surface.

In corresponding embodiments, the electrode device may be readily removed from a surrounding medium by releasing the compressive force along the longitudinal direction. The electrode device may, for example, be reused thereafter by mounting it in a different surrounding medium or at another position in the same surrounding medium.

Moreover, in such embodiments, the transverse distance may be reduced by releasing the compressive force along the longitudinal direction. This may be desirable to ensure an optimal electric contact between the electrically conductive surface and the surrounding medium, for example if a size of an opening in the surrounding medium that the electrode device is inserted into changes. The change may be related to a shrinkage or a swelling of the surrounding medium due to a change of its moisture content.

The elastic element(s) and/or the deformable electrode element(s) may comprise or may be composed of an elastic polymer.

In some embodiments, the elastic polymer is electrically conductive or comprises an electrically conductive section at the electrically conductive surface or at the electrically conductive surfaces. The elastic element(s) may be arranged between the rod and the electrically conductive section providing the electrically conductive surface.

The electrically conductive section and/or the elastic element and/or the deformable electrode element may comprises or be composed of a silicone. In particular, the electrically conductive section and/or the elastic element and/or the deformable electrode element may comprise more than 50 weight percent of silicone material, in particular more than 70 weight percent of silicone material or more than 80 weight percent of silicone material.

Corresponding embodiments may facilitate an elastic deformable electrode element and/or provide the advantages described in the context thereof.

The first terminal element and the second terminal element may further be adapted to release at least a portion of the compressive force along the longitudinal direction to contract the deformable electrode element(s) along a transverse direction perpendicular to the longitudinal direction and reduce the transverse distance of the electrically conductive surface(s) of the deformable electrode element(s) from the rod.

Corresponding first and second terminal elements may further contribute to the technical effects and advantages described in the context of the elastic deformable electrode element.

Each deformable electrode element may be adapted to expand along the transverse direction, when the compressive force is applied along the longitudinal direction, without forming cracks or breaking.

Each deformable electrode element may be adapted to expand along the transverse direction, when the compressive force along the longitudinal direction is applied, to increase the transverse distance of its electrically conductive surface from the rod by at least 5% of a transverse width of the insulation element, in particular by at least 10% of the transverse width of the insulation element or by at least 15% of the transverse width of the insulation element; in particular without forming cracks or breaking.

The deformable electrode element may comprise or consist of an electrically conductive elastomer. Alternatively, or in addition, the deformable electrode element may comprise or consist of an elastomer with the electrically conductive surface.

A transverse extension (width, or diameter) of the electrode device in a section between the first terminal element and the second terminal element may be at most 5 cm, in particular at most 3 cm or at most 2 cm, in particular in a state wherein no compressive force is applied along the longitudinal direction.

A corresponding extension of the electrode device along the transverse direction may facilitate inserting the electrode device into a bore hole with a corresponding or slightly larger diameter. Such a diameter of the bore hole may be sufficiently small to avoid affecting the structural integrity of the surrounding medium significantly. Also, the diameter may be sufficiently small for the bore hole to be generated with a commonly available drill.

An extension along the longitudinal direction of the section between the first terminal element and the second terminal element may be at least 1 cm or at least 3 cm.

The electrode device according to a corresponding embodiment may be suitable to measure a bulk moisture content of the surrounding medium rather than a surface moisture content, the latter being strongly affected by enhanced drying at the surface.

The electrode device may further comprise at least one terminal insulation element arranged between the deformable electrode element(s) and the first terminal element and/or the second terminal element, in particular, wherein the first terminal element and/or the second terminal element comprises an electrically conductive material and/or the rod comprises an electrically conductive material.

The at least one terminal insulation element may comprise or be composed of an electrically insulating material and/or be adapted to electrically insulate the deformable electrode element(s) and the first terminal element and/or the second terminal element.

The at least one terminal insulation element may allow for using electrically conductive material, such as steel, for the first terminal element and/or the second terminal element. The electrically conductive material may have preferable mechanical properties, for example for machining or in terms of lifetime, as compared to electrically insulating material such as a polymer or ceramics.

Each deformable electrode element of the pair of (neighboring) deformable electrode elements may be spaced by at least 1 cm from the first terminal element or from the second terminal element and/or from the other deformable electrode element of the pair of (neighboring) deformable electrode elements.

In corresponding embodiments, the pair of neighboring deformable electrode elements may be used to measure the moisture content of the surrounding medium at a sufficient depth to avoid exposed regions at the surface.

A transverse width of the first terminal element and/or of a terminal insulation element arranged between the deformable electrode element(s) and the first terminal element may exceed a transverse width of the second terminal element, transverse widths of any of the deformable electrode elements, transverse widths of any of the insulation elements, and/or a transverse width of a terminal insulation element arranged between the deformable electrode element(s) and the second terminal element.

A corresponding first terminal element or terminal insulation element may serve as a stopper and/or define a depth to which the electrode device is inserted into the surrounding medium.

The elastic element may comprise a material composition different from a material composition of the electrically conductive surface. The elastic element may be insulating.

Using different material compositions of the elastic element and of the electrically conductive surface may give additional freedom to optimize the corresponding material compositions.

For example, the material of the elastic element may comprise or be an elastic polymer to facilitate an elastic deformable electrode element.

For example, the material of the electrically conductive surface may comprise or be carbon or gold, for example in the shape of a foil or a coating, to minimize an electrical resistivity and/or to maximize a corrosion resistance.

In particular, a surrounding medium (construction material) such as wood may be corrosive and dissolve conductive materials conventionally used for electrodes, such as copper or silver. This may not only lead to a damage of the electrode, but also may change (lower) the resistivity of the surrounding medium in a vicinity of the electrode, affecting a resistive moisture measurement. The improved corrosion resistance of carbon or gold may avoid these problems.

Alternatively, the deformable electrode element(s) may comprise a homogeneous material composition.

The homogeneous material composition of the deformable electrode element(s) may facilitate the fabrication of the deformable electrode element, for example from an electrically conductive elastic polymer such as an electrically conductive silicone.

The homogeneous material composition may comprise a homogeneous electrically conductive material.

The electrically conductive surface(s) may comprise or be composed of an elastic polymer, in particular of an electrically conductive elastic polymer.

The electrically conductive surface(s) may comprise a plurality of electrically conductive particles, in particular electrically conductive particles comprising or composed of a noble metal, black carbon, or graphitic material, in particular graphitic material coated with a metal such as nickel.

The electrode device may further comprise metallic leads, wherein each metallic lead is in electrical contact with at least one electrically conductive surface and extends along the transverse direction beyond a volume defined by the deformable electrode elements and the insulation elements.

The metallic leads may comprise rounded metallic sections extending around the rod. The rounded metallic sections may be electrically insulated from the rod and may be in electrical contact with a section (sections) of the metallic leads, which extend(s) along the transverse direction beyond the volume defined by the deformable electrode elements and the insulation elements.

The rounded metallic sections may extend around the rod with an annular or ring-like shape.

The rounded metallic sections may be in electrical contact and optionally in direct physical contact with the deformable electrode element(s) and/or the electrically conductive surface.

The electrode device may further comprise a deformable sealing element arranged on the rod along the longitudinal direction between the first terminal element and the second terminal element, wherein the deformable sealing element is adapted to, when the compressive force is applied along the longitudinal direction, deform to generate an air-tight seal between the sealing element and a medium surrounding the electrode device.

The deformable sealing element may prevent moisture from escaping from the surrounding medium whose resistivity is to be measured, and therefore may avoid systematic errors in the measured moisture content related to the escaped moisture, such as a systematically smaller measured moisture content as compared to a bulk moisture content of the surrounding medium.

The deformable sealing element may have a circular or annular cross section.

The deformable sealing element may be adapted to, when the compressive force is applied along the longitudinal direction, compress along the longitudinal direction and/or expand along the transverse direction to generate the air-tight seal between the sealing element and the medium surrounding the electrode device.

The deformable sealing element may be characterized by one or all the features described above in the context of the deformable electrode elements.

The deformable sealing element may be one of the deformable electrode elements. In particular, the deformable sealing element may be a deformable electrode element closest to the first terminal element or a deformable electrode element closest to the second terminal element.

In other words, at least one of the deformable electrode elements may act as the deformable sealing element for sealing an interior of a bore hole in the medium surrounding the electrode device as a result of its transverse expansion.

The electrode device may further comprise at least one temperature sensor. The at least one temperature sensor may be adapted to measure a temperature of the surrounding medium.

The at least one temperature sensor may be arranged in a vicinity of the second terminal element, in particular on (an outer face of) an insulation element closest to the second terminal element or on a second terminal insulation element.

The at least one temperature sensor may be arranged along the longitudinal direction between the first terminal element and the second terminal element.

The at least one temperature sensor may measure a temperature, which may be used in relating the resistance between the pair of the deformable electrode elements to the moisture content of the surrounding medium and improve the accuracy of the process.

The at least one temperature sensor may be arranged on at least one of the insulation elements, on the rod or on at least one of the deformable electrode elements, in particular on an outer or inner face of the at least one of the insulation elements or of the at least one of the deformable electrode elements. The electrode device may further comprise at least one insulation structure arranged between the rod and the deformable electrode element and/or the electrically conductive surface. The at least one insulation structure may be adapted to electrically insulate the deformable electrode element and/or the electrically conductive surface from the rod.

The rod may comprise an electrically conductive material.

The insulation structure may be in direct physical contact with the deformable electrode element(s). The insulation structure may be adapted to define a lateral position of the deformable electrode element(s) and/or to prevent a movement of the deformable electrode element(s) along the transverse direction.

The deformable electrode element(s) may essentially encircle the insulation structure in a transverse plane. The insulation structure may essentially encircle the rod in a transverse plane.

The insulation structure may comprise an electrically insulating material and/or may be adapted to electrically insulate the electrode element(s) and/or the electrically conductive surface(s) from the rod.

The insulation structure may comprise a hollow section extending along the longitudinal direction. The hollow section may house at least a section of the metallic leads.

The rounded metallic sections of the metallic leads may extend around the insulation structure, in particular with an annular or ring-like shape.

The insulation structure may comprise a grove, in particular on its outer surface, wherein a portion of the rounded metallic sections of the metallic leads is arranged. In particular, the insulation structure may comprise at least one grove associated with each of the rounded metallic sections, wherein a portion of the associated rounded metallic section is arranged.

The at least one insulation structure may be arranged between the rod and each deformable electrode element, in particular between the rod and the electrically conductive surface of each of the deformable electrode elements.

The rod may comprise an electrically conductive material.

The at least one insulation structure may allow for using electrically conductive material, such as steel, for the rod. The electrically conductive material may have preferable mechanical properties, for example for machining or in terms of lifetime, as compared to electrically insulating material such as a polymer.

A system may comprise the electrode device and a calibration table adapted to relate a resistance between a pair of the deformable electrode elements to the resistivity of the surrounding medium and/or to a moisture content of the surrounding medium. In particular, the system may comprise machine-readable instructions comprising the calibration table. For example, the system may comprise a measurement device adapted to measure the resistance between the pair of neighboring deformable electrode elements and to relate the measured resistance to the moisture content of the surrounding medium using the calibration table and/or the machine-readable instructions.

The well-defined positions of the deformable electrode elements along the longitudinal direction may permit to determine the resistivity of the surrounding medium and/or the moisture of the surrounding medium based on the resistance between the pair of the deformable electrode elements using a simple, static calibration table instead of a more complex calibration model with a plurality of parameters, for example related to a variable arrangement of electrodes, for example in terms of lateral spacing or depth to which they are inserted into the surrounding medium.

The calibration table may be adapted to relate resistances between at least two different pairs of the deformable electrode elements to resistivities of the surrounding medium and/or to moistures of the surrounding medium.

The resistivities of the surrounding medium and/or the moistures of the surrounding medium may be adapted to determine a depth profile or a depth gradient of a moisture of the surrounding medium.

In some embodiments, the electrode device comprises at least three deformable electrode elements, in particular at least four deformable electrode elements or at least five deformable electrode elements.

Accordingly, resistivities between pairs of neighboring electrode elements, in particular neighboring deformable electrode elements may be used to estimate a gradient of the moisture content of the surrounding medium.

The electrode device may comprise a plurality of temperature sensors. For each pair of neighboring deformable electrode elements a temperature sensor of the plurality of temperature sensors may be provided. For each pair of neighboring deformable electrode elements, a temperature sensor may be arranged on the insulation element between the pair of neighboring deformable electrode elements.

In a second aspect, a method is provided for mounting an electrode device in a surrounding medium. The electrode device comprises a rod, a first terminal element, a second terminal element, and a plurality of electrode elements. The rod extends along a longitudinal direction. The first terminal element and the second terminal element are arranged on the rod at different positions along the longitudinal direction. The electrode elements are electrically insulated from each other. The plurality of electrode elements comprises a deformable electrode element. The deformable electrode element is arranged on the rod at a position along the longitudinal direction between the first terminal element and the second terminal element. The deformable electrode element comprises an electrically conductive surface facing away from the rod and with a transverse distance from the rod. The method comprises: Applying, using the first terminal element and the second terminal element, a compressive force along the longitudinal direction to the deformable electrode element; and thereby increasing the transverse distance of the electrically conductive surface of the deformable electrode element from the rod to generate an electric contact between the electrically conductive surface of the deformable electrode element and the surrounding medium.

The electrode device of the method may be characterized by features corresponding to the ones described above.

The method may further comprise, upon applying the compressive force along the longitudinal direction, compressing the deformable electrode element along the longitudinal direction and expanding the deformable electrode element along the transverse direction.

The method may further comprise, prior to applying the compressive force along the longitudinal direction, inserting the deformable electrode element or the deformable electrode elements or the electrode device into an opening of the surrounding medium.

The method may further comprise, prior to inserting the deformable electrode element or the deformable electrode elements or the electrode device into the opening of the surrounding medium, generating the opening of the surrounding medium, in particular drilling into the surrounding medium to generate the opening of the surrounding medium.

The increase of the transverse distance of the electrically conductive surface from the rod may further comprise establishing an electrical contact between the electrically conductive surface and the surrounding medium and/or the increase of the transverse distance of the electrically conductive surface from the rod may reduce an electrical contact resistance between the electrically conductive surface and the surrounding medium.

The electrode device may comprise a plurality of electrode elements comprising the deformable electrode element, and the method may further comprise measuring a resistance between a pair of electrode elements.

The electrode device may comprise a plurality of deformable electrode elements comprising the deformable electrode element, and the method may further comprise measuring a resistance between a pair of deformable electrode elements, in particular measuring resistances between at least two different pairs of the deformable electrode elements.

The method may further comprise, prior to measuring the resistance between the pair of the (deformable) electrode elements, adjusting the compressive force along the longitudinal direction for establishing an electrical contact between the pair of the deformable electrode elements and the surrounding medium.

The electrode device may further comprise metallic leads, wherein each metallic lead is in electrical contact with at least one electrically conductive surface and extends transversely beyond a volume defined by the deformable electrode elements and the insulation elements; and the method may further comprise measuring a resistance between a pair of the metallic leads, in particular measuring resistances between at least two different pairs of the metallic leads.

The surrounding medium may comprise or be a construction material, such as wood, concrete, or brick.

The method may further comprise reducing the compressive force along the longitudinal direction, thereby releasing the deformable electrode element along the transverse direction, and reducing the transverse distance of the section of the electrically conductive surface from the rod. The method may further comprise removing the electrode device from the opening of the surrounding medium.

The electrode device may further comprise a deformable sealing element arranged on the rod along the longitudinal direction between the first terminal element and the second terminal element. The method may further comprise, upon applying the compressive force along the longitudinal direction: Compressing the deformable sealing element along the longitudinal direction, and expanding the deformable sealing element along a transverse direction perpendicular to the longitudinal direction to generate an air-tight seal between the deformable sealing element and the medium surrounding the electrode device.

The air-tight seal may be between the deformable electrode element or at least one or all of the deformable electrode elements and the opening of the surrounding medium.

The sealing element may be the deformable electrode element or one of the deformable electrode elements, in particular a deformable electrode element of the deformable electrode elements closest to an open end of the opening of the surrounding medium.

The air-tight seal may prevent moisture from escaping from the opening. Therefore, systematic errors in the measured moisture content of the surrounding medium may be reduced, in particular a reduced measured moisture content as a result of a drying of a surface of the opening.

Alternatively, or in addition, the first terminal element may comprise a ring-shaped sealing face. Alternatively, the electrode device may further comprise a terminal insulation element arranged between the deformable electrode element(s) and the first terminal element and the terminal insulation element may comprise the ring-shaped sealing face. The ring-shaped sealing face may be pointing towards the second terminal element. The ring-shaped sealing face may encircle a cross section of the pair of deformable electrode elements.

The method may comprise providing a ring-shaped seal between the ring-shaped sealing face and the surrounding medium prior to inserting the electrode device into the opening of the surrounding medium. The method may further comprise, upon inserting the electrode device into the opening of the surrounding medium, generating an air-tight seal using the ring-shaped sealing face and the ring-shaped seal.

The method may further comprise measuring resistances between a pair of the deformable electrode elements and determining a moisture content of the surrounding medium based on the resistance between the pair of the deformable electrode elements.

In particular, the method may comprise measuring resistances between at least two different pairs of the deformable electrode elements and determining a moisture content of the surrounding medium based on the resistances between the at least two different pairs of the deformable electrode elements. The pairs of the deformable electrode elements may be pairs of neighboring deformable electrode elements.

The method may further comprise determining a depth profile or a gradient of a moisture content of the surrounding medium based on the measured resistances.

The electrode device may further comprise at least one temperature sensor arranged along the longitudinal direction between the first terminal element and the second terminal element. The method may further comprise determining a temperature by the at least one temperature sensor; and determining the moisture content of the surrounding medium based on the resistance between the pair of the deformable electrode elements and on the temperature determined by the at least one temperature sensor.

In particular, the method may comprise estimating a gradient of the moisture content of the surrounding medium based on the resistances between at least two different pairs of the deformable electrode elements and on the temperature determined by the at least one temperature sensor.

### BRIEF DESCRIPTION OF THE FIGURES

The techniques of the present disclosure and the advantages associated therewith will be best apparent from a description of exemplary embodiments in accordance with the accompanying drawings, in which:
- Fig. 1a: shows an electrode device according to an embodiment;
- Fig. 1b: shows an electrode device according to another embodiment;
- Fig. 2: shows an electrode device according to yet another embodiment;
- Fig. 3a: shows an electrode device inserted into a surrounding medium;
- Fig. 3b: shows a cross section of an electrode device inserted into a surrounding medium; and
- Fig. 4: illustrates a method for mounting an electrode device in a surrounding medium.

### DETAILED DESCRIPTION OF EMBODIMENTS

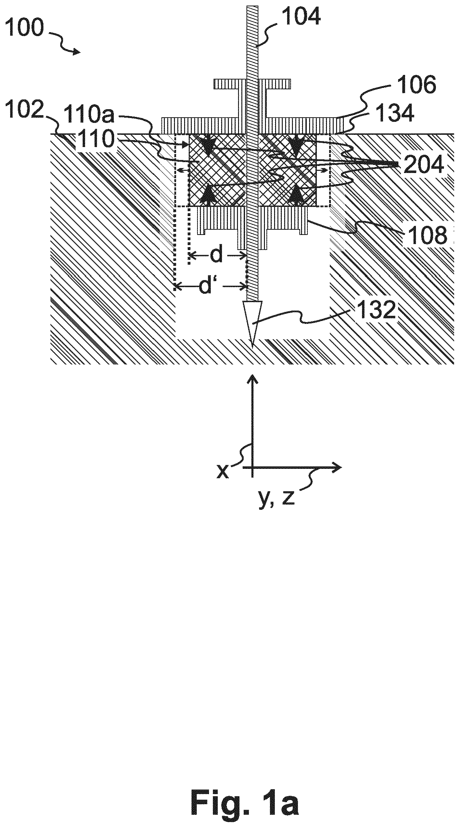

Fig. 1a is a schematic illustration of an electrode device 100 for measuring a resistivity of a surrounding medium 102.

According to the depicted embodiment, a central rod 104 supports all other components of the electrode device 100. In particular, terminal elements 106, 108 are mounted to the rod 104 and clamp a deformable electrode element 110.

The deformable electrode element 110 has at least one electrically conductive surface 110a initially at a distance d from the rod 104. The distance d refers to a distance in a transverse plane y, z perpendicular to the longitudinal direction x. In other words, the distance d refers to a distance along a transverse direction perpendicular to the longitudinal direction x, for example along (or between) one of the directions y, z. It is therefore referred to as a transverse distance d.

According to the depicted embodiment, the deformable electrode element 110 and its electrically conductive surface 110a are formed from a silicone-based material, more specifically silicone or fluorosilicone, doped with carbidic particles (black carbon or nickel-doped graphite) for electrical conductivity.

Silicone or fluorosilicone is deformable and elastic, i. e. recovers its initial shape when a force is applied and released. Silicone, and even more so fluorosilicone, is chemically inert and therefore provides a long lifetime for the deformable electrode element 110 and hence of the electrode device 100, even when permanently inserted into a construction material or in a construction site or outdoor environment. This is particularly advantageous when the electrode element 110 is to be inserted into a corrosive construction material such as wood or concrete. These construction materials hold the potential to chemically decompose conventional (metal) electrodes formed, for example, from copper or silver. This does not only damage the electrode device 100, but also results in undesirable uptake of metal ions in the construction material. These problems can be avoided using (fluoro)silicone-based electrodes.

30 - 60 (15 - 30) weight percent of the carbidic particles have been added to the fluorosilicone (silicone) to provide a suitable conductivity. While other species such as noble metal particles may be added to the silicone for conductivity, the use of carbidic particles further suppresses the corrosion described above and improves the lifetime of the deformable electrode element 110 further.

The (fluoro)silicone with the carbidic particles was shaped into a plate, and deformable electrode elements 110 were punched out of the plate. Alternatively, the silicon doped with the carbidic particles was shaped into the hollow cylindrical shape of the deformable electrode element 110 using a suitable mold.

The first terminal element 106 is mounted to the rod 104 movably along the longitudinal direction x. The moveable mounting may be implemented by connecting the first terminal element 106 to the rod 104 using a threading, gearing, or clamping mechanism.

According to the depicted embodiment, the second terminal element 108 is mounted to the rod 104 in the same way as the first terminal element 106. In embodiments, wherein the second terminal element 108 comprises a nut, an additional nut may be used to counter the second terminal element 108 and fix its position on the rod 104. Alternatively, the second terminal element 108 may be fixed to the rod 104 with a permanent joint, for example by welding or staking.

Applying a force to the first terminal element 106 directed at moving it closer to the second terminal element 108 generates a compressive force 204 onto the deformable electrode element 110.

The deformation of the deformable electrode element 110 caused by said compressive force 204 results in an increase of the initial transverse distance d between the electrically conductive surface 110a and the rod 104 to a larger transverse distance d' between the electrically conductive surface 110a and the rod 104. More specifically, the deformable electrode element 110 is compressed along the longitudinal direction x and expands in the transverse plane y, z while essentially maintaining its volume.

As a consequence of its larger transverse distance d' from the rod 104, the electrically conductive surface 110a may electrically contact the surrounding medium 102 next to/around the deformable electrode element 110. In particular, the electrode device 100 may be inserted into a borehole in a construction material such that the construction material encircles the electrode device 100 in a vicinity of the deformable electrode element 110 and hence forms the surrounding medium 102. When the initial transverse distance d is enlarged, the electrically conductive surface 110a electrically contacts the surrounding medium 102.

After the electrical contact has been established between the electrically conductive surface 110a and the surrounding medium 102, a resistivity of the surrounding medium 102 may be determined by measuring an electrical resistance between the electrically conductive surface 110a and a second electrode (not shown). The second electrode may in principle be a conventional electrode or another electrode device 100 as described above. However, the second electrode is preferably a further electrically conductive portion of the electrode device 100, such as an electrically conductive section 132 at or close to the lower end of the rod 104 or an electrically conductive section 134 of the first terminal element 106, which may be electrically insulated from the deformable electrode element 110 by an insulating spacer (not shown). From the measured electrical resistance or the determined resistivity, a moisture content of the surrounding medium 102 may be determined using a reference table accounting for a material composition of the surrounding medium 102, a temperature of the surrounding medium 102, and a distance between the second electrode and the electrically conductive surface 110a.

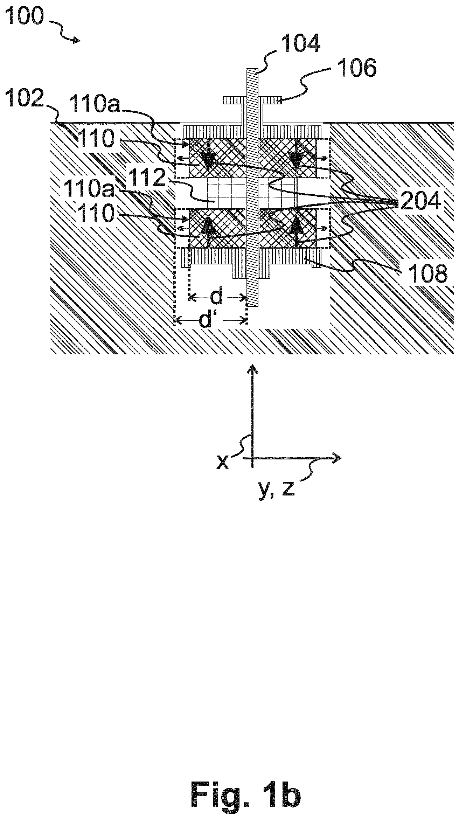

Fig. 1b shows an electrode device 100 according to a modified embodiment. This embodiment is similar to the one described in the context of Fig. 1a. Similar elements have same reference numbers and have been described above.

As compared to the embodiment of Fig. 1a, the embodiment of Fig. 1b is modified in that it comprises two deformable electrode elements 110 and an insulation element 112. The deformable electrode elements 110 are spaced along the longitudinal direction x, and the insulation element 112 is located between the deformable electrode elements 110. The terminal elements 106, 108 are mounted to the rod 104 and clamp the deformable electrode elements 110 as well as the insulation element 112. Applying a force to the first terminal element 106 directed at moving it closer to the second terminal element 108 generates a compressive force 204 onto the deformable electrode elements 110 and onto the insulation element 112.

The material compositions of the deformable electrode elements 110 and the insulation element 112 are selected such that deformable electrode elements 110 are more deformable than the insulation element 112. For example, the deformable electrode elements 110 have a smaller Young's modulus than the insulation element 112, or undergo a larger deformation when exposed to a compressive mechanical force, respectively. According to the depicted embodiment, the insulation element 112 consists of polytetrafluoroethylene (Teflon) which is less deformable than the silicone of the deformable electrode elements 110.

Hence, the compressive force 204 results in a deformation of the deformable electrode elements 110, whereas the insulation element 112 essentially maintains its shape.

After the electrical contact has been established between the electrically conductive surfaces 110a and the surrounding medium 102 by applying the compressive force 204, a resistivity of the surrounding medium 102 may be determined by measuring an electrical resistance between the electrically conductive surfaces 110a. From the measured electrical resistance or the determined resistivity, a moisture content of the surrounding medium 102 may be determined using a reference table accounting for a material composition of the surrounding medium 102, a temperature of the surrounding medium 102, and a distance between the electrically conductive surfaces 110a.

To ensure that the measured electrical resistance is not affected by parasitic shunts between the electrically conductive surfaces 110a through the electrode device 100 (in particular through the rod 104), the electrically conductive surfaces 110a are electrically insulated from each other within the electrode device 100. Therefore, according to an embodiment, the rod 104 is fabricated from an electrically insulating material, for example from a polymer or a ceramic. Alternatively, only outer material sections, e.g. only at the conductive surfaces 110a, of the deformable electrode elements 110 are doped or coated with the carbidic particles, whereas the rest of the deformable electrode elements 110 is made from an electrically insulating (fluoro)silicone.

The electrode device 100 is fabricated by first providing the rod 104 and the terminal elements 106, 108. Corresponding parts are readily available, for example in the form of a threaded rod 104 with a diameter of 4 mm and matching nuts 106, 108. The insulation element 112 made from polytetrafluoroethylene in the shape of a cylinder, for example with an outer diameter of 20 mm, a height of 10 mm, and an inner diameter matched to the diameter of the rod 104, may be machined from a larger piece by turning a full cylinder and drilling a center hole.

Thereafter, the second terminal element 108, the first deformable electrode element 110, the insulation element 112, the second deformable electrode element 110 and the first terminal element 106 are arranged on the rod 104. The second terminal element 108 and the first terminal element 106 are mounted to the rod 104 as described above, for example using the threaded rod 104 and the matching nuts 106, 108, to clamp the deformable electrode elements 110 and the insulation element 112 to their respective positions on the rod between the first terminal element 106 and the second terminal element 108, thereby completing the electrode device 100.

The electrode device 100 of Fig. 1a can be fabricated accordingly, but with only one deformable electrode element 110 and without the insulation element 112.

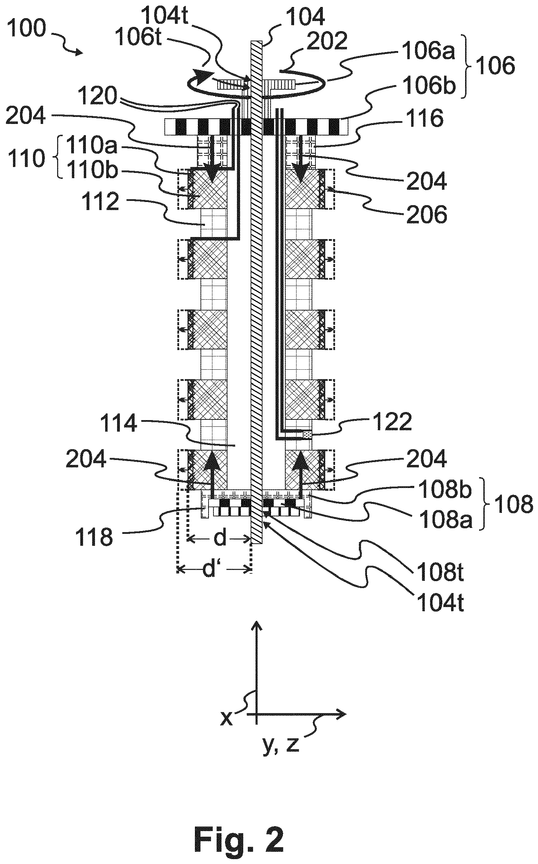

Fig. 2 shows an electrode device 100 according to a different embodiment. This embodiment is similar to the one described in the context of Fig. 1b. Similar elements have same reference numbers and have been described above.

The embodiment of Fig. 2 comprises a series of modifications, which will subsequently be laid out in detail. According to different embodiments, the electrode device 100 may be formed with a single one or any combination of these modifications.

The electrode device 100 of Fig. 2 comprises five deformable electrode elements 110 and four insulation elements 112, each arranged between a pair of neighboring deformable electrode elements 110. Depending on the material composition and the thickness of the surrounding medium to be monitored and the depth range of interest, the electrode device 100 may be formed with a suitable number of deformable electrode elements 110 and insulation elements 112, for example with three deformable electrode elements 110 and two insulation elements 112, with nine deformable electrode elements 110 and eight insulation elements 112, and so on.

The electrode device 100 of Fig. 2 further comprises an insulation structure 114 in the form of a hollow tube arranged between the rod 104 and the deformable electrode elements 110. The insulation structure 114 is made from an electrically insulating material, for example polytetrafluoroethylene, to electrically insulate the deformable electrode elements 110 from the rod 104. Therefore, the insulation structure 114 prevents parasitic electric shunts between the deformable electrode elements 110 and the rod 104. Therefore, a rod 104 made from materials with arbitrary electric properties may be used without risking to shunt different conducting surfaces 110a. The rod 104 of the embodiment of Fig. 2 is made from stainless steel.

The electrode device 100 of Fig. 2 further comprises metallic leads 120, each connected to a conducting surface 110a. The metallic leads 120 are guided inside the insulation structure 114. The insulation structure 114 has a suitable (sufficient) inner diameter to provide enough room for the metallic leads 120. The metallic leads 120 are each surrounded by a cable sheath (not shown) with a resistance exceeding the resistance of the construction material of which the moisture content is to be measured. The construction materials, such as wood, concrete, or brick, whose moisture content is to be measured, have large resistances. Therefore, the cable sheaths have significant thicknesses. A hollow tube with an inner diameter of 8 mm, encircling a rod 104 with a diameter of 4 mm, has proven suitable to provide enough room for nine metallic leads 120 with respective cable sheaths.

The metallic leads 120 extend laterally/transversely beyond any of the deformable electrode elements 110, insulating elements 112, and beyond the first terminal element 106 to facilitate measuring a resistance between a pair of conducting surfaces 110a. Only two metallic leads 120 are depicted in the figure, but preferably one metallic lead 120 is provided per conducting surface 110a and is in electric contact with the respective deformable electrode element 110 and (thereby with) its conducting surface 110a.

The metallic leads 120 of the electrode device 100 of Fig. 2 comprise metallic rings arranged on (groves in) the outer face of the hollow tube constituting the insulation structure 114, at positions corresponding to the positions of the deformable electrode elements 110 . Along the longitudinal direction x, the metallic leads 120 are guided as wires through the insulation structure 114. At the position of the metallic rings, the wires are guided through openings in the insulation structure 114, e.g. grooves or through-bores, to contact the metallic rings and hence the deformable electrode elements 110.

Alternatively, or in addition, leads 120 may also be arranged on the outer face of the insulation structure 114 and may be formed by electrically conductive sections on the outside of the insulation structure 114, e.g. partially covered by an insulating layer for electrically contacting only selected deformable electrode elements 110 in a pre-defined area of the insulation structure 114 along the longitudinal direction.

The electrode device 100 of Fig. 2 further comprises a first terminal insulation element 116 arranged between the first terminal element 106 and an uppermost deformable electrode element 110. The first terminal insulation element 116 helps to avoid parasitic electrical shunts between the uppermost deformable electrode element 110 and the first terminal element 106. The first terminal element 106 may consequently be formed from a material with arbitrary electrical properties, including a metal. The first terminal element 106 of Fig. 2 is made from stainless steel. The electrode device 100 of Fig. 2 similarly comprises a second terminal insulation element 118 arranged between the second terminal element 108 and a lowermost deformable electrode element 110 to electrically insulate the two from each other. The second terminal element 108 may consequently be formed from a metal, and is composed of stainless steel according to the embodiment of Fig. 2.

According to the embodiment of Fig. 2, the rod 104 comprises a thread 104t.

According to the embodiment of Fig. 2, the first terminal element 106 comprises a nut 106a with a counter-thread 106t to the thread 104t and a washer 106b. The first terminal element 106 is mounted to the threaded rod 104 movably along the longitudinal direction x. In particular, the first terminal element 106 is movable by turning 202 the nut 106a with the counter-thread io6t on the thread 104t of the rod 104. Therefore, the thread 104t and the counter-thread io6t mediate the compressive force 204. The washer 106b improves the homogeneity of the compressive force 204.

According to the embodiment of Fig. 2, the second terminal element 108 comprises a nut 108a with a counter-thread io8t to the thread 104t and a washer 108b. The second terminal element 108 is mounted to the threaded rod 104 movably along the longitudinal direction x. In particular, the second terminal element 108 is movable by turning the nut 108a with the counter-thread io8t on the thread 104t of the rod 104. Therefore, the thread 104t and the counter-thread io8t mediate the compressive force 204. The washer 108b improves the homogeneity of the compressive force 204. Alternatively, the second terminal element 108 may be fixed to the rod 104 with a permanent joint, for example by welding or staking.

According to the embodiment of Fig. 2, the deformable electrode elements 110 each consist of an elastic element 110b and the electrically conductive surface 110a. A material composition of the elastic element 110b is selected for the elastic element 110b to be more deformable than the insulation element 112, as described above in the context of the deformable electrode elements 110. The electrically conductive surface 110a is formed from an electrically conductive material. According to a preferred embodiment, the elastic element 110b and the electrically conductive surface 110a have identical material compositions and form an integral piece. According to the depicted embodiments, both are composed of fluorosilicone doped with carbidic particles as described above.

According to alternative embodiments, the elastic element 110b is formed from undoped and electrically insulating silicone. The electrically conductive surface 110a may be doped or may be formed by an electrically conductive coating or an electrically conductive foil, preferable a slitted electrically conductive foil such as a slitted, gold-plated foil, on the elastic element 110b.

The electrode device 100 of Fig. 2 further comprises a temperature sensor 122 for measuring a temperature at the position of the temperature sensor 122. The measured temperature may be used for determining the moisture content of the surrounding medium as described above. The depicted temperature sensor 122 is arranged on an outer surface of an insulation element 112 located between a pair of neighboring electrically conductive surfaces 110a or deformable electrode elements 110, respectively. In Fig. 2, a single temperature sensor 122 is depicted, but a temperature sensor 122 may be provided on each insulation element 112, in particular on the outer surface of each insulation element 112, and may be associated with a pair of neighboring electrically conductive surfaces 110a or deformable electrode elements 110, respectively. Therefore, a locally measured temperature may be associated with any pair of neighboring electrically conductive surfaces 110a, or any resistance measurement between a pair of neighboring electrically conductive surfaces 110a, respectively. Therefore, the moisture content of the surrounding medium may be measured locally between any a pair of neighboring electrically conductive surfaces 110a accounting for the associated, locally measured temperature.

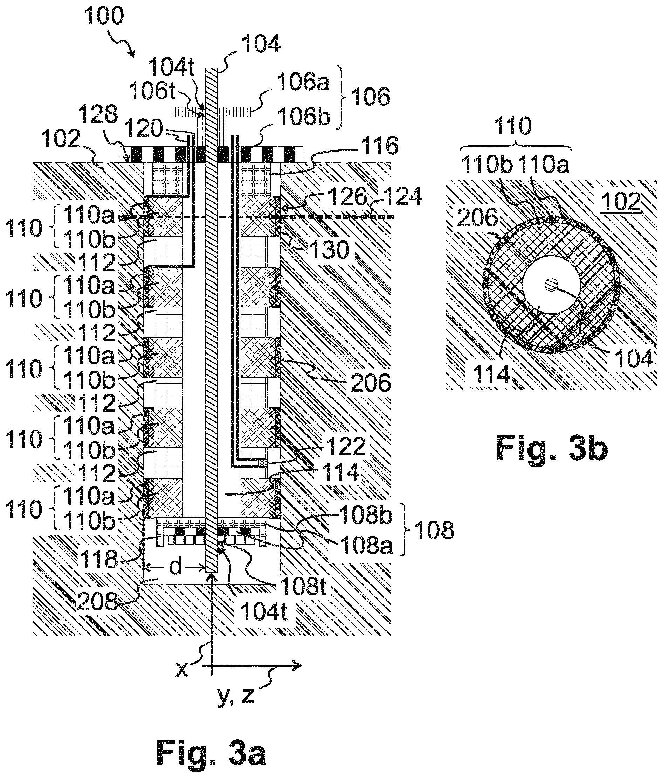

Fig. 3a and Fig. 3b show an electrode device 100 inserted into a surrounding medium 102. According to the depicted embodiment, the surrounding medium 102 is a construction material such as wood or concrete. The electrode device 100 is similar to the one described in the context of Fig. 2. Similar elements have similar reference numbers and have been described above.

The embodiment of Fig. 3a and Fig. 3b comprises a series of modifications related to mounting the electrode device in the surrounding medium, which will be described below in detail. According to different embodiments, the electrode device 100 may be formed with a single one or any combination of these modifications. Moreover, process steps for mounting the electrode device 100 in the surrounding medium 102 will be described. A method for mounting the electrode device 100 in the surrounding medium 102 may comprise any combination of these process steps.

To mount the electrode device 100 in the surrounding medium 102, an opening 208 is drilled in the surrounding medium 102. The opening has a width, or diameter, respectively.

The width, or diameter, respectively, of the opening 208, is selected to be larger than the widths of the second terminal element 108, the second terminal insulation element 118, the deformable electrode elements 110, the insulation elements 112, the rod 104 and the insulation structure 114. The width, or diameter, respectively, of the opening 208, is selected to be smaller than the width of the first terminal element 106, in particular of the washer 106b. According to the depicted embodiment, the width, or diameter, respectively, of the opening 208 is larger than the width of the first insulation element 116, but according to alternative embodiments, the width, or diameter, respectively, of the opening 208 is smaller than the width of the first insulation element 116.

The depth of the opening 208 is selected to be as large or larger than a length of the electrode device 100 from the first terminal element 106 to a far end of the electrode device 100 from the first terminal element 106.

Then, the electrode device 100 is inserted into the opening 208. More specifically, any element of the electrode device 100 with a width smaller than the width, or diameter, respectively, of the opening 208 is inserted into the opening 208. Any element of the electrode device 100 with a width larger than the width, or diameter, respectively, of the opening 208 remains outside of the opening 208. The first terminal element 106 with its width exceeding the one of the opening 208 serves as a stopper to determine the depth to which the electrode device 100 is inserted into the opening 208. In alternative embodiments (not shown), the first terminal insulation element 116 has a width exceeding the one of the opening 208 and serves as the stopper to determine the depth to which the electrode device 100 is inserted into the opening 208. The contact area 128 between the corresponding stopper 106 serves as a ring-shaped sealing face 128 to seal the opening 208 from the environment, preferably in a moisture-proof manner. The sealing may further be improved by providing a sealing ring (not shown) on the ring-shaped sealing face 128 prior to inserting the electrode device 100 into the opening 208.

Then, the nut 106a of the first terminal element 106 is tightened on the thread 104t of the rod 104 to generate the compressive force 204 along the longitudinal direction x onto the deformable electrode elements 110 and the transverse expansion 206 of the deformable electrode elements 110 in the transverse plane y, z. The transverse distance d of the electrically conductive surfaces 110a is increased to the larger transverse distance d' at which the electrically conductive surfaces 110a are in direct physical contact and in electric contact with the surrounding medium 102.

According to alternative embodiments, and as depicted, for example, in Fig. 1b, the first terminal element 106 is also at least partially inserted into the surrounding medium 102. In such embodiments, an elongated wrench or key, for example in the shape of a hollow tube with a structure along its inner surface, may be used to tighten the first terminal element 106 located (at least partially) inserted into the surrounding medium 102.

Thereafter, the nut 106a of the first terminal element 106 is tightened on the thread 104t of the rod 104 even harder to generate a bias compressive force onto the deformable electrode elements 110. The additional tightening is preferably performed with a defined torque, for example using a torque wrench. The bias force further expands the deformable electrode elements 110 along the transverse plane y, z in case of an increase of the width, or the diameter, respectively, of the opening 208. A change of the width, or the diameter, respectively, of the opening 208 may be the result of a shrinkage or expansion of the surrounding medium, for example due to a change of its moisture content. If an increase of the width, or diameter, respectively, of the opening 208 occurs, the bias compressive force acts to enlarge the transverse distance d' of the electrically conductive surfaces 110a from the rod 104 correspondingly and ensures the direct physical contact and electric contact between the electrically conductive surfaces 110a and the surrounding medium 102.

According to the embodiment depicted in Fig. 3a and Fig. 3b, the uppermost deformable electrode element 110 acts as a sealing element 130 to generate an air-tight seal 126 between its outermost surface and the surrounding medium 102. To illustrate the air-tight seal 126 in more detail, Fig. 3b depicts a cross section through the electrode device 100 and the surrounding medium 102 along the reference plane 124 of Fig. 3a. As the outermost surface 110a of the uppermost deformable electrode element 110 is in direct physical contact with the surrounding medium 102, it seals the section of the opening 208 below the direct physical contact from the environment above the surrounding medium 102 and acts as the air-tight seal 126. According to the depicted embodiment, the uppermost deformable electrode element 110 serves as the sealing element 130. In alternative embodiments (not shown), a sealing element 130 with similar mechanical properties as the uppermost deformable electrode element 110 may be provided, however, without providing an electrical conductivity and therefore without acting as an electrode element.

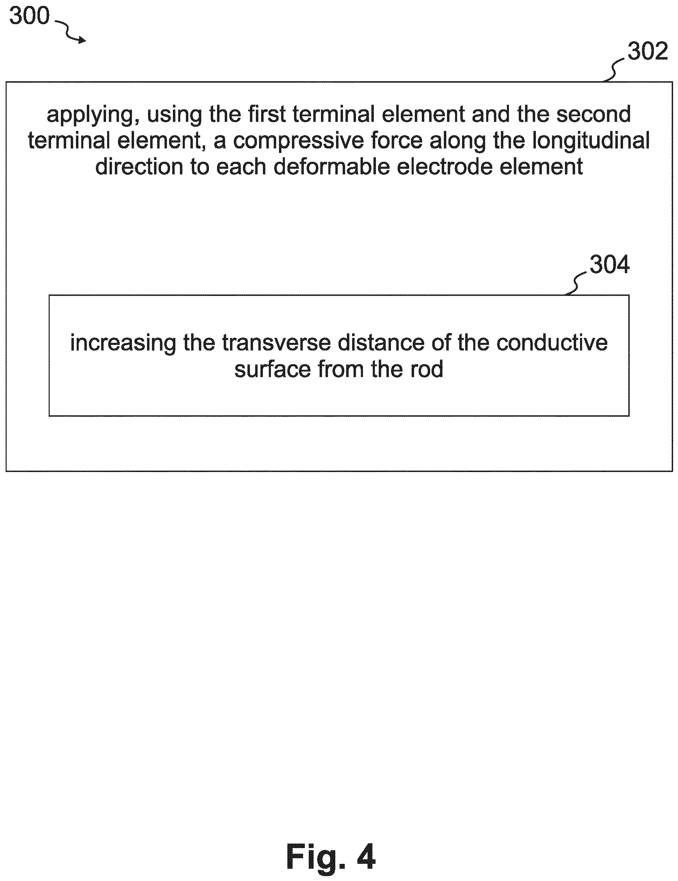

Fig. 4 summarizes the most import process steps of a method 300 for mounting an electrode device 100 in a surrounding medium 102.

In step 302, a compressive force 204 along the longitudinal direction x is applied to each deformable electrode element 110 using the first terminal element 106 and the second terminal element 108.

In step 304, a transverse distance d of the electrically conductive surface 110a from the rod 104 is thereby increased.

### LIST OF REFERENCE SIGNS

- 100: electrode device
- 102: surrounding medium
- 104: rod
- 104t: thread of rod
- 106: first terminal element
- 108: second terminal element
- 106a: knurled nut
- 108a: nut (low form)
- 106b, 108b: washer
- io6t, io8t: counter-thread
- 110: deformable electrode elements
- 110a: electrically conductive surface
- 110b: elastic element
- 112: insulation element
- 114: tubular insulation structure
- 116: first terminal insulation element
- 118: second terminal insulation element
- 120: metallic leads
- 122: temperature sensor
- 124: reference plane
- 126: air-tight seal
- 128: ring-shaped sealing face
- 130: sealing element
- 132,134: electrically conductive section
- d, d': transverse distance of the electrically conductive surface from the rod
- x: longitudinal direction
- (y, z): plane perpendicular to the longitudinal direction
- y, z: transverse direction
- 202: turning the nut of the first terminal element
- 204: compressive force along the longitudinal direction
- 206: transverse expansion

## Claims

1. An electrode device (100) for measuring a resistivity of a surrounding medium (102), the electrode device (100) comprising:
a rod (104) elongated along a longitudinal direction (x);
a first terminal element (106, 108) and a second terminal element (108, 106) arranged on the rod (104) at different positions along the longitudinal direction (x); and
a plurality of electrode elements (110, 132, 134), wherein the electrode elements (110, 132, 134) are electrically insulated from each other;
wherein the plurality of electrode elements (110, 132, 134) comprises a deformable electrode element (110) arranged on the rod (104) at a position along the longitudinal direction (x) between the first terminal element (106, 108) and the second terminal element (108, 106), wherein the deformable electrode element (110) comprises an electrically conductive surface (110a) facing away from the rod (104), with a transverse distance (d, d') from the rod (104); and
wherein the first terminal element (106, 108) and the second terminal element (108, 106) are adapted to apply a compressive force (204) along the longitudinal direction (x) to the deformable electrode element (110) to increase the transverse distance (d, d') of the electrically conductive surface (110a) of the deformable electrode element (110) from the rod (104).

2. The electrode device (100) according to claim 1,
wherein the plurality of electrode elements (110, 132, 134) comprises a plurality of deformable electrode elements (110) arranged on the rod (104) at different positions along the longitudinal direction (x) between the first terminal element (106, 108) and the second terminal element (108, 106), wherein each deformable electrode element (110) of the plurality of deformable electrode elements (110) comprises an electrically conductive surface (110a) facing away from the rod (104), with a transverse distance (d, d') from the rod; and
wherein the electrode device (100) comprises an insulation element (112) arranged between a pair of deformable electrode elements (110) of the plurality of deformable electrode elements (110) and adapted to electrically insulate the deformable electrode elements (110) of the pair from each other.

3. The electrode device according to claim 2, comprising a plurality of insulation elements (112) comprising the insulation element (112), wherein each insulation element (122) of the plurality of insulation elements (112) is arranged between a pair of deformable electrode elements (110) and adapted to electrically insulate the deformable electrode elements (110) of the pair from each other.

4. The electrode device (100) according to claim 2 or 3, wherein the deformable electrode element (110) is adapted to provide a larger deformation than the insulation element (112) when the compressive force (204) is applied along the longitudinal direction (x).

5. The electrode device (100) according to any of the preceding claims, wherein the deformable electrode element (110) comprises or is composed of an elastic polymer.

6. The electrode device (100) according to any of the preceding claims, wherein the first terminal element (106, 108) and the second terminal element (108, 106) are further adapted to release at least a portion of the compressive force (204) along the longitudinal direction (x) to contract the deformable electrode element (110) along a transverse direction (y, z) perpendicular to the longitudinal direction (x) and reduce the transverse distance (d, d') of the electrically conductive surface (110a) of the deformable electrode element (110) from the rod (104).

7. The electrode device (100) according to any of the preceding claims,
wherein the deformable electrode element (110) comprises or consists of an electrically conductive elastomer; and/or
wherein the deformable electrode element (110) comprises or consists of an elastomer with the electrically conductive surface (110a).

8. The electrode device (100) according to any of the preceding claims, which further comprises a deformable sealing element (130) arranged on the rod (104) along the longitudinal direction (x) between the first terminal element (106, 108) and the second terminal element (108, 106), wherein the deformable sealing element (130) is adapted to, when the compressive force (204) is applied along the longitudinal direction (x), deform to generate an air-tight seal (126) between the deformable sealing element (130) and a medium surrounding the electrode device (100).

9. The electrode device (100) according to any of the preceding claims, which further comprises at least one temperature sensor (122) adapted to measure a temperature of the surrounding medium (102).

10. The electrode device (100) according to any of the preceding claims comprising at least one insulation structure (114),
wherein the at least one insulation structure (114) is arranged between the rod (104) and the electrically conductive surface (110a) and is adapted to electrically insulate the electrically conductive surface (110a) from the rod (104).

11. A method (300) for mounting an electrode device (100) in a surrounding medium (102), the electrode device (100) comprising:
a rod (104) extending along a longitudinal direction (x);
a first terminal element (106, 108) and a second terminal element (108, 106) arranged on the rod (104) at different positions along the longitudinal direction (x); and
a plurality of electrode elements (110, 132, 134), wherein the electrode elements (110, 132, 134) are electrically insulated from each other;
wherein the plurality of electrode elements (110, 132, 134) comprises a deformable electrode element (110) arranged on the rod (104) at a position along the longitudinal direction (x) between the first terminal element (106, 108) and the second terminal element (108, 106), wherein the deformable electrode element (110) comprises an electrically conductive surface (110a) facing away from the rod (104) with a transverse distance (d, d') from the rod (104);
wherein the method comprises:
applying (302), using the first terminal element (106, 108) and the second terminal element (108, 106), a compressive force (204) along the longitudinal direction (x) to the deformable electrode element (110); and thereby
increasing (304) the transverse distance (d, d') of the electrically conductive surface (110a) of the deformable electrode element (110) from the rod (104) to generate an electric contact between the electrically conductive surface (110a) of the deformable electrode element (110) and the surrounding medium (102).

12. The method (300) according to claim 11, which further comprises, prior to the application (302) of the compressive force (204) along the longitudinal direction (x), inserting the deformable electrode element (110) into an opening of the surrounding medium (102).

13. The method (300) according to claim 11 or claim 12, wherein the electrode device further comprises a deformable sealing element (130) arranged on the rod (104) along the longitudinal direction (x) between the first terminal element (106, 108) and the second terminal element (108, 106), and wherein the method further comprises, upon applying (302) the compressive force (204) along the longitudinal direction (x):
compressing the deformable sealing element (130) along the longitudinal direction (x); and
expanding (206) the deformable sealing element (130) along a transverse direction (y, z) perpendicular to the longitudinal direction (x);
to generate an air-tight seal (126) between the deformable sealing element (130) and the medium (102) surrounding the electrode device (100).

14. The method (300) according to claim 12 or 13, wherein the electrode device (100) comprises a plurality of deformable electrode elements (110) comprising the deformable electrode element, and wherein the method further comprises measuring a resistance between a pair of the deformable electrode elements (110) and determining a moisture content of the surrounding medium (102) based on the resistance between the pair of the deformable electrode elements (110).

15. The method (300) according to claim 14, wherein the electrode device (100) further comprises at least one temperature sensor (122) arranged along the longitudinal direction (x) between the first terminal element (106, 108) and the second terminal element (108, 106), and wherein the method further comprises:
determining a temperature by the at least one temperature sensor (122); and
determining the moisture content of the surrounding medium (102) based on the resistance between the pair of the deformable electrode elements (110) and on the temperature determined by the at least one temperature sensor (122).
